# EUROPEAN PATENT APPLICATION

(11) **EP 3 900 723 A1**
(43) Date of publication of application: **27.10.2021**
(21) Application number: 20315196.4
(22) Date of filing: 20.04.2020
(51) Int. Cl.: A61K 31/465, A61P 31/14, A61K 9/70

(54) **NACHR, SUCH AS NICOTINE, FOR USE IN THE PREVENTION AND THE TREATMENT OF COVID-19 DISEASE**

(71) Applicant: SORBONNE UNIVERSITE, 75006 Paris (FR); Institut Pasteur, 75015 Paris (FR); ASSISTANCE PUBLIQUE, HOPITAUX DE PARIS, 75004 Paris (FR)
(72) Inventor: Miyara, Makoto, 75013 Paris (FR); Amoura, Zahir, 94170 LE Perreux SUR Marne (FR); Tubach, Florence, 75013 Paris (FR); REY, Felix Augusto, 75015 Paris (FR); Changeux, Jean-Pierre, 75015 Paris (FR)
(74) Representative: Regimbeau

(57) **Abstract**

The invention relates to a ligand having a modulatory effect on neuronal nicotinic acetylcholine receptor (nAChR) or a pharmaceutical salt thereof for use for preventing and/or treating viral infections due to at least one betacoronavirus, preferably a Severe Acute Respiratory Syndrome-related coronavirus.

SEQUENCE LISTING

<110> SORBONNE UNIVERSITE
INSTITUT PASTEUR
ASSISTANCE PUBLIQUE - HOPITAUX DE PARIS

<120> PREVENTION AND TREATMENT OF COVID-19 DISEASE

<130> B76031EP D40559

<160> 6

<170> PatentIn version 3.5

<210> 1
<211> 12
<212> PRT
<213> SARS-CoV-2 virus

<400> 1

Tyr Gln Thr Gln Thr Asn Ser Pro Arg Arg Ala Arg
1               5                   10

<210> 2
<211> 11
<212> PRT
<213> serpentes

<400> 2

Cys Asp Gly Phe Cys Ser Ser Arg Gly Lys Arg
1               5                   10

<210> 3
<211> 11
<212> PRT
<213> rabies virus

<400> 3

Cys Asp Ile Phe Thr Asn Ser Arg Gly Lys Arg
1               5                   10

<210> 4
<211> 11
<212> PRT
<213> rabies virus

<400> 4

Cys Asp Ile Phe Thr Asn Ser Arg Gly Lys Arg
1               5                   10

<210> 5
<211> 11
<212> PRT
<213> rabies virus

<400> 5

Cys Asp Ile Phe Thr Asn Ser Asp Gly Lys Arg
1               5                   10

<210> 6
<211> 11
<212> PRT
<213> serpentes

<400> 6

Cys Asp Ala Phe Cys Ser Ser Arg Gly Lys Val
1               5                   10

## Description

### FIELD OF THE INVENTION

The present invention relates to a new therapeutic or veterinary use of a known compound, for preventing and/or treating viral infections caused by at least one Betacoronavirus, in particular by the SARS-CoV-2 (Severe Acute Respiratory Syndrome Coronavirus 2), this viral infection in humans being designated as COVID-19 disease.

### BACKGROUND OF THE INVENTION

SARS-CoV-2 belongs to the species *Coronavirus,* in the genus *Betacoronavirus* and family *Coronaviridae.*

Coronaviruses are enveloped viruses with a helically symmetrical capsid. They have a single-stranded, positive-sense RNA genome and are capable of infecting cells in birds and mammals. The morphology of the virions is typical, with a halo of protein protuberances ('Spike') which gave them their name of 'crown virus'.

Among the four genera of coronaviruses, the Betacoronavirus genus (B-CoVs or Beta-CoVs), comprising virus infecting animals and/or humans, is subdivided into four lineages designated as A, B, C and D:
- Lineage A also designated as subgenus *Embecovirus* includes HCoV-OC43 and HCoV-HKU1, virus able to infect various species;
- Lineage B also designated as subgenus *Sarbecovirus* includes SARS-CoV-1, SARS-CoV-2, and Bat SL-CoV-WIV1;
- Lineage C also designated as subgenus *Merbecovirus* includes Tylonycteris bat coronavirus HKU4 (BtCoV-HKU4), Pipistrellus bat coronavirus HKU5 (BtCoV-HKU5), and MERS-CoV, able to infect notably camels and humans;
- Lineage D also designated as subgenus *Nobecovirus* includes Rousettus bat coronavirus HKU9 (BtCoV-HKU9).

The Betacoronaviruses of the greatest clinical importance concerning humans are:
- OC43 and HKU1 of the A lineage,
- SARS-CoV-1 and SARS-CoV-2 of the B lineage, and
- MERS-CoV of the C lineage.

In humans, coronavirus infections can cause respiratory pathologies associated with symptoms similar to the common cold, bronchiolitis and more serious diseases such as the Severe Acute Respiratory Syndrome caused by SARS-CoV-1, which generated an epidemic in 2003, and the Middle Eastern Respiratory Syndrome caused by MERS-CoV, which generated an epidemic in 2012.

SARS-CoV-2 is the betacoronavirus causing the coronavirus epidemic of 2019-2020, generating the form of pneumonia known as coronavirus disease 2019 or COVID-19.

According to the World Health Organization (WHO), by April 5, 2020, 1 133 758 cases of COVID-19 have been confirmed and 62 784 patients have died worldwide. This epidemic was declared a public health emergency of international concern by WHO on January 30, 2020.

SARS-CoV-2 genome was sequenced for the first time on 5 January 2020 by a team from Fudan University in Shanghai (China). Its genome consists of a single-stranded RNA of 29,903 nucleotides. It is 79.5% similar to that of SARS-CoV-1, and 96% similar to that of bat coronavirus, suggesting that the origin of the SARS-CoV-2 virus is zoonotic and is found in bats (Zhou P et al. A pneumonia outbreak associated with a new coronavirus of probable bat origin. Nature. 2020).

Symptoms of infection with SARS-CoV-2 are roughly similar to those of seasonal influenza infections: they include fever, fatigue, dry cough, shortness of breath, difficult breathing, pneumonia, renal failure, and may lead to death in severe cases. Recently, neurologic and psychiatric manifestations have been described.

The severity of clinical signs requires that approximately 20% of patients remain in hospital and 5% require admission to intensive care. WHO estimates the mortality rate to be around 2% (data of February 2020). However, this case-fatality rate remains uncertain, due to the difficulty in estimating the number of confirmed cases and deaths directly attributable to SARS-CoV-2 infection in the field.

The most serious forms are observed in people who are vulnerable because of their age (over 70) -but these forms can also be observed in younger patients- or because of associated diseases such as hypertension, diabetes, obesity and/or coronary heart disease.

It is of the most importance to identify therapeutic compounds for preventing and/or treating COVID-19 disease. Indeed, today, no specific therapy or vaccine is currently available for the prevention or treatment of SARS-CoV-2 viral infection.

The majority of treatments currently received by COVID-19 patients are primarily aimed at alleviating the symptoms of fever, cough and dyspnea in order to promote spontaneous recovery.

Antiviral compounds, known for their therapeutic activity on other types of viruses, are currently tested in clinical trials. The French High Council for Public Health issued an opinion on March 23, 2020 on the therapeutic recommendations for the management of COVID-19 disease. This official notice proposes in particular to use the following therapeutic compounds: Remdesivir, Lopinavir/Rotinavir and Hydroxychloroquine.

Remdesivir has been initially developed for the treatment of Ebola virus infections. Remdesivir is a broad-spectrum antiviral compound, acting as a nucleoside analogue, specifically an adenosine analogue. Its presence misleads the viral polymerase and causes a reduction in viral RNA synthesis.

Lopinavir is a viral protease inhibitor, previously used against the human immunodeficiency virus (HIV). Lopinavir inhibits the production of functional proteins by the new virions, thereby blocking the spread of the virus. Lopinavir is rapidly degraded in the body. For this reason, it is administered in fixed combination with Ritonavir, which inhibit cytochrome P450 monooxygenases, thereby slowing the degradation of Lopinavir by these enzymes.

Hydroxychloroquine, initially known for its anti-malaria activity, has been shown to have an apparent efficacy in the treatment of Covid-19 (Yao et al., 2020). However, clinical data are still limited.

There is a pressing urgency to identify preventive and therapeutic treatments based upon the presently available scientific information. In particular, identification of biological targets of the SARS-CoV-2 might allow to define novel therapeutic strategies.

The following clinical symptoms of COVID-19 disease are of special interest for understanding the physiology of this viral infection:
- neurological/psychiatric disorders have been notified, especially the loss of sense of smell;
- in hospitalized patients with bad prognosis, the following symptoms have been noted:
   ∘ systemic hyperinflammatory syndrome, with increased levels of circulating pro-inflammatory cytokines, and
   ∘ atypical acute respiratory distress syndrome, with loss of neurological control of lung perfusion regulation, and hypoxic vasoconstriction.

Therefore, there is strong evidence for a neurotropic action of SARS-CoV-2 infection.

It has been demonstrated that B-coronaviruses do not limit their presence to the respiratory tract and frequently invade the central nervous system. This propensity has been convincingly documented for the SARS-CoV-1 and MERS-CoV. In light of the high similarity between SARS-CoV-1 and SARS-CoV-2, it is quite likely that SARS-CoV-2 also possesses a similar potential. Neuro-infection potentially contributes to clinical manifestations of COVID-19, and the neuro-invasive potential of SARS-CoV-2 could play a role in the respiratory failure of COVID-19 patients.

As mentioned, loss of sense of smell frequently occurs in COVID-19 patients. Furthermore, several studies have reported that some patients infected with SARS-CoV-2 show neurologic signs such as headache, nausea and vomiting. A study on 214 COVID-19 patients further found that about 88% among the severe patients displayed neurologic manifestations including acute cerebrovascular diseases and impaired consciousness.

Based on these data, it is now admitted that SARS-CoV-2 virus enters the nervous system, invades the brain stem and affects the medullary neurons of the respiratory centers. Further studies have been performed to understand the physiological mechanisms of such central nervous system invasion.

The angiotensin converting enzyme ACE2 represents the principal receptor molecule for cell entry of SARS-CoV-2 (Lan, J. et al. Structure of the SARS-CoV-2 spike receptor-binding domain bound to the ACE2 receptor. Nature, 2020). The ACE2 is expressed in the brain, in both neurons and glia. Nevertheless, additional receptors or co-receptors for the entry of SARS-CoV-2 are not excluded.

### SUMMARY OF THE INVENTION

A first object of the present invention is a ligand having a modulatory effect on neuronal nicotinic acetylcholine receptor (nAChR) or a pharmaceutical salt thereof for use for preventing and/or treating viral infections due to at least one betacoronavirus, preferably a Severe Acute Respiratory Syndrome-related coronavirus (SARS-CoV), in particular SARS-CoV-2.

A second object of the present invention is a transdermal delivery device comprising a ligand having a modulatory effect on neuronal nicotinic acetylcholine receptor (nAChR) or a pharmaceutically acceptable salt thereof for use for preventing and/or treating viral infections due to at least one betacoronavirus, advantageously a Severe Acute Respiratory Syndrome-related coronavirus, in particular SARS-CoV-2.

This effector ligand of neuronal nicotinic acetylcholine receptor (nAChR) is in particular nicotine.

### BRIEF DESCRIPTION OF THE FIGURES

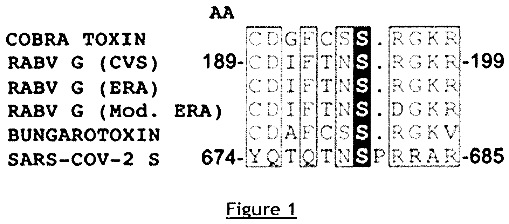

**Figure 1** discloses the amino acid sequence alignment of the motifs found in toxins from snakes of the Ophiophagus (cobra) and Bungarus genera, in G from three RABV strains and in S from SARS-CoV-2.

### DEFINITIONS

In the context of the invention, the term "betacoronavirus" designates any virus belonging to the Betacoronavirus genus (B-CoVs or Beta-CoVs), in particular any betacoronavirus belonging to one of the four lineages designated as A, B, C and D. It designates a betacoronavirus infecting animals and/or humans. In particular, this designation includes the betacoronaviruses infecting human organisms chosen among OC43, HKU1, SARS-CoV-1, SARS-CoV-2 and MERS-CoV.

"Viral infections due to at least one betacoronavirus" designates the fact that host cells of an organism have been infected by at least one betacoronavirus, the whole organism being said to have a viral infection.

A betacoronavirus infection in humans is usually diagnosed by a healthcare professional, based on observation of the infected patient's symptoms. Additional biological tests may be required to confirm the diagnosis: blood and/or sputum and/or bronchoalveolar fluid tests. Infection by a betacoronavirus can be established, for example, by molecular biological detection and/or viral titration of respiratory specimens, or by assaying blood for antibodies specific for said betacoronavirus. Conventional diagnostic methods comprise techniques of molecular biology such as PCR, which is well known to the person in the field.

In the sense of the invention, the term "prevention" means preventing, or reducing the likelihood of the occurrence of, an infection in a human or animal organism by at least one coronavirus. By administering the compound for its use according to the invention, human or animal cells of said organism become less permissive to infection, and are thus more likely to be resistant to infection by said betacoronavirus.

The term "treatment" refers to fighting the betacoronavirus infection in a human or animal organism. By administering the compound used according to the invention, the rate of viral infection (infectious titer) in the organism will decrease, and preferably the betacoronavirus will completely disappear from the organism within a shorter period of time than expected without treatment. The term "treatment" also refers to reducing the symptoms associated with the betacoronavirus infection (respiratory syndrome, kidney failure, fever, etc.).

A ligand having a modulatory effect on neuronal nicotinic acetylcholine receptor (nAChR), in particular nicotine, for its use according to the invention may be intended to be administered to a human being or to non-human animals.

In the context of the invention, the terms "COVID-19 disease" mean the disease linked to the infection with the SARS-CoV-2 (Severe Acute Respiratory Syndrome Coronavirus 2).

In the context of the invention, the "symptomatic COVID-19 disease" is characterized by a patient who shows at least one symptom of the COVID-19 disease. The most common symptoms of the COVID-19 disease are fever, muscle aches, headaches, fatigue, loss of taste and smell and respiratory symptoms such as a dry cough, difficulty breathing and a lack of oxygen. A symptomatic disease is in contrast to an asymptomatic disease which is characterized by a patient who is a carrier for a disease or infection but experiences no symptoms.

In the context of the invention, the "severe symptomatic COVID-19 disease" is characterized by severe symptoms of the COVID-19 disease, in particular respiratory symptoms. Severe symptoms are acute respiratory distress syndrome and/or hyper-inflammatory state that require hospitalization of the patient, especially in the intensive care unit (ICU).

In the context of the invention, "COVID-19 patients" are patients infected with the SARS-CoV-2 (Severe Acute Respiratory Syndrome Coronavirus 2).

In the context of the invention, a "transdermal delivery device" is an adhesive device which can administer, for medical purposes, an active principle through the skin. Such a device provides direct administration of active principle to the blood system without passing by the digestive system, as is the case with tablets, capsules or syrups.

In the context of the invention, the abbreviation "LATA" designates the phase of "Limitation and discontinuation of active therapies" where palliative care begins, to accompany patients at the end of life.

The abbreviation "ICU" refers to an intensive care unit, a special department of a hospital or health care facility that provides intensive treatment medicine.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt" is intended to mean, in the framework of the present invention, a salt of a compound which is pharmaceutically acceptable, as defined above, and which possesses the pharmacological activity of the corresponding compound.

The pharmaceutically acceptable salts comprise:
1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
2) base addition salts formed when an acid proton present in the compound is either replaced by a metal ion, such as an alkali metal ion, an alkaline-earth metal ion, or an aluminum ion; or coordinated with an organic or inorganic base. Acceptable organic bases comprise diethanolamine, ethanolamine, N-methylglucamine, triethanolamine, tromethamine and the like. Acceptable inorganic bases comprise aluminum hydroxide, calcium hydroxide, potassium hydroxide, sodium carbonate and sodium hydroxide.

### DETAILED DESCRIPTION OF EMBODIMENTS OF THE INVENTION

In contrast with prior art studies showing that lung is the primary target of the SARS-COV-2 virus, and that ACE2 is the principal receptor of COVID-19 for its entry in cells, the epidemiological evidence and the *in silico* findings of the present inventors suggest that the COVID-19 infection is a nAChR disease that could be prevented and controlled by a ligand having a modulatory effect on nAChR, in particular nicotine.

More precisely, the present inventors postulated that such ligand of nAChR can sterically (or allosterically) compete with the SARS-CoV-2 binding to at least one of the nAChRs, thereby preventing or reducing the hyperinflammatory syndrome that may be lethal for the patient. To confirm this hypothesis, the present inventors have analyzed for the first time the smoking habits of patients that have been diagnosed from being infected by SARS-CoV-2. Severe cases ("inpatients") and non-severe cases ("outpatients") have been included and followed.

Their results show for the first time that, compared to the French general population, the COVID-19 population exhibited a significantly decreased current daily smoker rate by 80.3% for outpatients and by 73.9% for inpatients. Thus, current smoking status (i.e. consumption of nicotine) appears to be a protective factor against the infection by SARS-CoV-2.

In this context, they propose to administer a treatment based on a ligand having a modulatory effect on nAChR, and in particular a ligand mimicking the binding of nicotine to its receptor, in order to antagonize the blocking action of SARS-CoV-2 on the AChR, and prevent the subsequent deficit it causes in the Central Nervous System.

In the context of the invention, the terms "cerebral nicotinic acetylcholine receptor", "neuronal nicotinic acetylcholine receptor" and "nAChR" are synonymous and designate any type of oligomeric receptors able to bind acetylcholine and nicotine, specifically expressed in the peripheral and central nervous system of mammalians.

These receptors are pentameric structures, composed of combinations of five subunits. Twelve neuronal nAChR subunits have been described, designated as alpha2 to alpha10 and beta2 to beta4; they are differentially expressed throughout the nervous system and combine to form various nAChRs. The *α*4*β*2 and *α*7 nAChR oligomers are the two most frequent, accounting for about 95% of the whole nAChR population in the human brain.

These nAChRs are neurotransmitters whose conformation changes when allosteric interactions take place between topographically distinct sites, which leads to a reversible conformational change of the protein and modulates the opening of the central ion channel.

The endogenous ligand of these receptors is acetylcholine, but they are also reactive to different external ligands such as nicotine, alcohol and toxins.

In the context of the invention, the terms "ligand having a modulatory effect on neuronal nicotinic acetylcholine receptor (nAChR)", "nAChR effector ligand" and "nicotinic ligand", used interchangeably, designate any orthosteric and/or allosteric ligand of any nAChR, that bind to this nAChR and have a modulatory effect on this receptor. This term includes activators and inhibitors of nAChRs. It includes all nAChR agonists, partial agonists, positive allosteric modulators, antagonists, partial agonists, or negative allosteric modulators.

Several reviews cite these types of compounds, see for example (Mohamed TS, Jayakar SS, Hamouda AK. Orthosteric and Allosteric Ligands of Nicotinic Acetylcholine Receptors for Smoking Cessation. Front Mol Neurosci. 2015; 8:71. 2015 Nov 25.).

Naturally, numbers of these nicotinic ligands have been evaluated for their ability to help smokers to quit tobacco addiction, as nicotine replacement therapy. Among these nicotinic ligands, one can cite the following compounds: varenicline, cytisine, dianicline, ABT-418, ABT-089, CP-601927, and CP-601932.

Nicotinic acetylcholine receptor ligands have also been evaluated as potential therapeutic agents for cognitive disorders (Terry AV, Callahan PM. Nicotinic Acetylcholine Receptor Ligands, Cognitive Function, and Preclinical Approaches to Drug Discovery. Nicotine Tob Res. 2019;21(3):383-394).

In a specific embodiment of the invention, the considered nAChR effector ligand is nicotine. In another embodiment, the nAChR effector ligand is a nicotine derivative.

In another embodiment, the nAChR effector ligand is a monoclonal antibody specifically targeting at least one nAChR.

In another specific embodiment of the invention, the nAChR effector ligand is not ivermectin.

In a first aspect, the present invention relates to the use of at least one nAChR effector ligand or a pharmaceutical salt thereof to prepare a medicament intended to prevent and/or treat patients that have been diagnosed to be infected with at least one betacoronavirus. In other terms, the invention addresses at least one nAChR effector ligand or a pharmaceutical salt thereof, for use for preventing and/or treating viral infections due to at least one betacoronavirus in a subject suffering thereof.

The present invention also discloses a method for preventing or treating any subject that has been diagnosed to be infected with at least one betacoronavirus, said method comprising administering to said subject an effective amount of at least one nAChR effector ligand or of a pharmaceutical salt thereof. The method of the invention can comprise a diagnosis step as preliminary step.

The said infection can be diagnosed or detected by any conventional means, such as molecular biological detection and/or viral titration of respiratory specimens, or by assaying blood for antibodies specific for said betacoronavirus.

As used herein, the term "treatment of the invention" designates nAChR effector ligand or a pharmaceutical salt thereof, in all the embodiments disclosed below.

In a particular embodiment, said betacoronavirus is chosen in the group consisting of: OC43, HKU1, SARS-CoV-1, SARS-CoV-2 and MERS-CoV. In a more particular embodiment, said betacoronavirus is a Severe Acute Respiratory Syndrome-related coronavirus (SARS-CoV), and even more particularly, SARS-CoV-2.

In a preferred embodiment, said subject is a human patient. Said patient can be older than 75 years old, between 65 and 74 years old, between 15 and 44 years old, or below 15 years old. In a more preferred embodiment, said human patient is older than 65 years old, even more preferably older than 75 years old. These people indeed usually experience the most severe symptoms when infected with said virus.

Said subject can be a smoker or a non-smoker. Preferably, it is a non-smoker.

In a preferred embodiment, said subject is an animal chosen in the group consisting of: bats, hedgehog, camels, mice, humans, pigs, cats, among others. Any other animal that can be infected with a betacoronavirus could also benefit from the treatment of the invention.

In a particular embodiment, the treatment of the invention is administered to subjects (animals or humans) that have been diagnosed to be infected with at least one betacoronavirus but do not present any symptom yet. In another embodiment, the treatment of the invention is administered to subjects (animals or humans) that display at least one symptom of the COVID-19 disease, e.g., fever, muscle aches, headaches, fatigue, loss of taste, loss of smell or respiratory symptoms such as a dry cough. In another embodiment, the treatment of the invention is administered to subjects (animals or humans) that suffer from severe symptomatic COVID-19 disease and experience for example difficulty in breathing and/or a lack of oxygen.

Accordingly, the treatment of the invention is also useful to prevent and/or treat acute respiratory distress syndrome associated with betacoronavirus infection. The purpose of the treatment of the invention is to increase the survival rate of the infected subjects.

In a specific embodiment of the invention, at least one nAChR effector ligand is used, meaning that a combination of two, three, four or more nAChR effector ligands could be used for preventing and/or treating viral infections due to at least one betacoronavirus.

In a specific embodiment of the invention, at least one of the nAChR effector ligands is nicotine.

In another specific embodiment of the invention, the used nAChR effector ligand is nicotine.

In the context of the invention, the nicotine ((S)-3-(1-methyl-2-pyrrolidinyl)pyridine) may be in any forms such as a free base or a pharmaceutically acceptable salt such as a hydrochloride salt. Advantageously, the nicotine is in a free base form (hereinafter occasionally referred to as "nicotine free base").

In the context of the invention, the nAChR effector ligand, in particular nicotine, or the pharmaceutically acceptable salt thereof, may be administered via oral, parenteral, topical, transcutaneous, nasal or buccal route.

Advantageously, the nAChR effector ligand, in particular nicotine, or the pharmaceutically acceptable salt thereof, is administered via transcutaneous route.

Advantageously, the nAChR effector ligand, in particular nicotine, or the pharmaceutically acceptable salt thereof, is administered using a transdermal delivery device, more advantageously using a transdermal patch.

The transdermal delivery device or the patch uses the cutaneous pathway to provide direct administration of active principle to the blood system without passing by the digestive system.

Transdermal delivery devices, in particular transdermal patches, comprising at least one nAChR effector ligand, in particular nicotine, or a pharmaceutically acceptable salt thereof, are commonly known and marketed.

There are several types of patches which can be categorized (in *Topical Drug Bioavailability, Bioequivalence and Penetration,* V.P. Shah and H.I. Maibach, published by Springer, 1993, page 53) according to the mechanism by which the active principle is released and to the patch's design. Among the six categories listed, nicotine-based patches are those of categories II, IVa and IVc, alone or in combination. By directly incorporating nicotine in the adhesive, the type IVa patch has the advantage of having a small thickness and of being capable of being cut to size in this exceptional case by virtue of the homogeneous distribution of the active principle across its entire surface, unlike, for example, type II patches comprising a depot region.

Thus, in the context of the invention, the transdermal delivery device is advantageously type IVa patches.

Advantageously, the transdermal patch comprises a support layer, a self-adhesive matrix layer comprising the nAChR effector ligand, in particular nicotine, or the pharmaceutically acceptable salt thereof, and a detachable protective film.

By "support layer according to the invention" is meant any support layer typically used in the field of patches.

By "detachable protective film" is meant all detachable protective films capable of protecting the device's matrix before the device is used. These films typically used in the field of transdermal devices are well-known to the person skilled in the art.

The matrix layer according to the invention is self-adhesive. By "self-adhesive" is meant that the matrix layer is able to keep the device according to the invention bound to a support, for example the skin, in a stable manner, with no need for the use of other means of bonding. Preferentially, by "self-adhesive" is meant that the matrix layer allows the device to adhere to the skin for at least 12 hours, preferentially for about 12 to 48 hours, more preferentially for about 24 hours.

Advantageously, the transdermal delivery device according to the invention allow the release of a nAChR effector ligand, in particular nicotine, or a pharmaceutically acceptable salt thereof, from the matrix layer by an average delivery rate comprised between 5 and 100 µg/cm²/h, advantageously between 10 µg/cm²/h and 50 g/cm²/h, for a time interval comprised between 1 and 24 hours. The release rate may be measured by any permeation technique known to the person skilled in the art, in particular via permeation kinetics, for example on the abdominal skin of nude mouse arranged on the surface of a glass (Franz) cell.

Advantageously, the transdermal patch used in the context of the invention is the patch marketed as Nicopatch® or Nicopatchlib®.

Advantageously, the transdermal delivery device of the invention comprises between 5 mg and 30 mg of nicotine or a pharmaceutically acceptable salt thereof, advantageously between 10 mg and 25 mg, more advantageously between 14 mg and 25 mg, in particular 21 mg.

In another aspect of the invention, the nAChR effector ligand, in particular nicotine, or the pharmaceutically acceptable salt thereof, is administered via oral or buccal route, in particular buccal route. For example, the nAChR effector ligand, in particular nicotine, or the pharmaceutically acceptable salt thereof, is advantageously administered via buccal route using pharmaceutical sucking lozenges or sublingual tablets. In this context, the sublingual tablets or sucking lozenges comprise advantageously between 1 mg and 30 mg of nicotine or a pharmaceutically acceptable salt thereof, advantageously between 1 mg and 20 mg, more advantageously between 1 mg and 10 mg, in particular 2 mg. The pharmaceutical tablets or sucking lozenges can thus be administered several times per day.

Advantageously, in the context of the invention, a nAChR effector ligand, in particular nicotine, or pharmaceutically acceptable salt thereof, is administered in a dosage of between 5 and 30 mg per 24 hours, advantageously between 10 mg and 25 mg per 24 hours, more advantageously between 14 mg and 25 mg per 24 hours, in particular 21 mg per 24 hours.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### I. EPIDEMIOLOGIC STUDY ON COVID-19 INFECTED PATIENTS

### Patients and design

A cross sectional survey investigating the rate of current smokers in patients with a diagnosis of COVID-19 has been performed first in hospitalized patients (i.e., severe symptomatic cases of COVID-19) and second, in outpatients (i.e. the non-severe symptomatic cases of COVID-19). Current smoker rates were compared to those of the French population as reference, after standardization on age and sex.

Eligible patients were those with a confirmed diagnosis of COVID-19 at the APHP Pitié-Salpêtrière Hospital, Paris, France, either hospitalized in medical wards of medicine, but not in ICUs (inpatients) or having consulted for this infection in the infectious disease department and who did not require hospital care until the end of the acute infectious episode (outpatients). Data from inpatients, hospitalized from March 23 to April 9, 2020 and from outpatients, who consulted from February 28 to March 30, 2020 were collected. The present study is observational. All data were collected in the context of care, very few data were collected in completely anonymous sheets, thus, in accordance with the French law, including the GPRD, and informed consent of the patient was not sought. This study has been approved by the ethics committee of Sorbonne University (2020 - CER-2020-13).

### Definitions and data collected

Confirmed COVID-19 was defined as a positive result on real-time reverse-transcriptase-polymerase-chain-reaction (RT-PCR) assay of nasal and pharyngeal swab specimens (NPRS).

Smoking status was collected, patients were specifically asked if they were current smokers (and if so, if they smoked every day or occasionally), former smokers, or not smokers ever.

Daily smokers are individuals reporting daily smoking or reporting a daily frequency of the number of cigarettes (manufactured or rolled) or other tobacco products (cigar, cigarillos, pipe, shisha). Occasional smokers are individuals reporting smoking but not every day and only reporting no consumption of tobacco product per day. The group of ex-smokers includes anyone having smoked in the past, occasionally or daily, and who reported not smoking at the time of the investigation. Under the term "never smoker" people never smoked and those who just tried once or twice. The quantities of tobacco smoked have been calculated with the following equivalences: 1 cigar = 1 cigarillo = 2 cigarettes.

For current smokers the number of daily cigarettes was recorded.

In addition to smoking status, the following data were extracted from the medical records: Age class (by 10-year class), sex, comorbidities known to have potentially an impact on the prognosis of covid-19 infection (diabetes, hypertension, obesity, immunodepression, respiratory disease (COPD), clinical manifestations of COVID infection, out- or inpatient status.

### Smoking rate in the population of reference

The population of reference to compute Standardized incidence ratios is the French population. Recent rates of current daily smokers have been reported for year 2018 by sex and age class (of 10 years) from the General survey Baromètre de Santé publique France (Public Health Barometer), a cross sectional phone survey made yearly on representative sample of 18-75-year-old people living in mainland France, with a on 2-level random sampling (Andler, R. et al. Reduction of daily smoking rate among adults: results from the 2018 santé publique france health barometer. Bull Epidémiol Hebd 15, 271-277 (2019)). The 2018 survey involved a sample of 9,074 adults of 18-75-year-old. The completion of the survey took place from January 10 to July 25, 2018.

### Statistical analysis

A descriptive analysis has been made by group (inpatients - outpatients). Qualitative variables were described by numbers and percentages, and quantitative variables by median and interquartile range. Inpatients and outpatients were compared with Pearson Chi2 tests.

The standardized incidence ratios (SIR) were used to compare current daily smoker rates in the COVID19 inpatients and outpatients, respectively, to current daily smoker rates in a reference population, here the French general population in 2018. The SIR is the ratio of observed number of current daily smokers in the COVID19 patients to the number of current daily smokers that would be expected, on the basis of age- and gender-specific current daily smokers' rates, in the general population. The SIR was estimated and the 95% confidence interval calculated.

The main analysis involved all included patients, and those older than 75 years were considered in the 65-75 years age class for standardization (which is conservative for the outstanding hypothesis, as current smoker rates decrease with age). For 9 outpatients, medical charts were not available and thus smoking status was unavailable. These patients were not included in the main analysis (missing chart which were very likely to be at random). Two sensitivity analyses were performed, one excluding patients older than 75 years, another one considering the 9 patients with missing data on the smoking status as current smokers.

### Demographic and Clinical Characteristics

A total of 343 inpatients and 139 outpatients were included. The demographic and clinical characteristics of the two groups are shown in Table 1.

**TABLE 1 - Clinical characteristics and smoking habits of COVID-19 patients**

| | **Outpatients** | | | **Inpatients** | | | **Outpatient/in patient comparison** p value |
|---|---|---|---|---|---|---|---|
| | Male n(%) | Female n(%) | all | Male n(%) | Female n(%) | all | |
| **patients** | 64 (44.6) | 76(55.3) | 139 | 206 (60.1) | 137 (39.9) | 343 | 0.002 |
| **Median (IQR) age (yrs)** | 43 (32-55) | 44(32-54) | 43 (32-55) | 66 (54-76) | 65 (55-79) | 65 (54-77) | <0.0001 |
| **PCR +** | 64(100) | 76 (100) | 139 (100) | 206(100) | 137(100) | 343(100) | |

| **Coexisting disorder** | | | | | | | |
|---|---|---|---|---|---|---|---|
| **High pressure blood** | 9(15.25%) | 7(9.59%) | 16 (12.12%) | 85 (41.26%) | 57 (41.61%) | 142 (41.4%) | <0.0001 |
| **Diabetes** | 4(6.78%) | 3 (4.11%) | 7(5.3%) | 54 (26.21%) | 41 (29.93%) | 95 (27.7%) | <0.0001 |
| **Obesity** | 4(6.78%) | 6 (8.22%) | 10 (7.58%) | 29(14.57%) | 19(14.07%) | 48 (14.37%) | 0.0453 |
| **Immune deficiency** | 3 (5.08%) | 1 (1.37%) | 4 (3.03%) | 34(16.5%) | 27(19.71%) | 61(17.78%) | <0.0001 |
| **COPD** | 2(3.39%) | 0 (0%) | 2 (1.52%) | 18 (8.74%) | 9 (6.57%) | 27 (7.87%) | 0.0095 |

| **Smoking status** | | | | | | | |
|---|---|---|---|---|---|---|---|
| **Current active** | 3(5.08%) | 4(5.48%) | 7 (5.3%) | 11 (5.37%) | 4(2.94%) | 15(4.4%) | 0.6753 |
| **Current occasional** | 3(5.08%) | 3 (4.11%) | 6 (4.55%) | 5(2.44%) | 1 (0.74%) | 6 (1.76%) | 0.1034 |
| **Ever non smoker** | 21 (35.59%) | 21 (28.77%) | 42 (31.82%) | 78 (38.05%) | 34(25%) | 112 (32.84%) | 0.8308 |
| **Former** | 32 (54.24%) | 45 (61.64%) | 77 (58.33%) | 111 (54.15%) | 97 (71.32%) | 208(61%) | 0.5954 |
| **Missing** | 3 (4.84%) | 4 (3.9%) | 7 (5.04%) | 1 (0.49%) | 1 (0.73%) | 2 (0.58%) | |

The inpatient group was composed of 343 patients, median age 65 years: 206 men (60.1%, median age 66 years) and 137 women (39.9%, median age 65 years). The rate of daily smokers was 4.4 (5.4% of men and 2.9% of women).

The outpatient group was composed of 139 patients, median age 44 years: 62 men (44.6 %, median age 43 years, and 77 women (55.4 %, median age 44 years). The daily smokers' rate was 5.3% (5.1% of men and 5.5 % of women).

As described by others (Guan, W. J. et al. Clinical Characteristics of Coronavirus Disease 2019 in China. N Engl J Med, doi:10.1056/NEJMoa2002032 (2020)), hypertension (41.4%), diabetes (27.7%), obesity (14.4%) and immune deficiencies (17.8%) were frequently observed in inpatients while COPD was less frequent (7.9%). Those comorbidities were significantly less frequent in outpatients with hypertension reported in 12.1%, (P<0.0001), diabetes in 5.3% (P<0.0001), obesity in 7.6% (P=0.045), immunodeficiencies in 3.0 % (P<0.0001), and COPD in 1.5% (P= 0.009).

### Comparison with the French general population

The rate of daily current smokers in inpatients (4.4%) did not significantly differ from that in outpatients (5.4%; P= 0.67; TABLE 2). Occasional smoking was slightly more frequent in outpatients than in inpatients (4.6 vs 1.8 %; P=0.10) but the number of people concerned was small.

Standardized Incidence Ratio for daily current smokers according to sex and age is shown in Table 2. SIRs were 0.197 [0.094 - 0.414] and 0.246 [0.148 - 0.408] for outpatients and inpatients, respectively. The SIR in outpatients did not significantly differ from that in inpatients (p = 0.63). Sensitivity analyses yielded similar results (results hot shown).

**TABLE 2 - Standardized incidence ratio of current smokers out- and inpatients**

| | Outpatients | | | | | | | Inpatients | | | | | |
|---|---|---|---|---|---|---|---|---|---|---|---|---|---|
| Sex | Age class | Crude rate | Reference rate | CSR | Overall standardized rate | SIR | P | Crude rate | Reference rate | CSR | Overall standardized rate | SIR | P |
| | | | | | [IC 95%] | [IC 95%] | | | | | [IC 95%] | [IC 95%] | |
| Female | 18-24 | 0.0000 | 0.288 | 0.0153 | 26.87 [25.64 - 28.21] | 0.1973 [0.0941 - 0.4139] | <0.001 | 0.0000 | 0.288 | 0.0008 | 17.87 [16.93 - 18.77] | 0.2462 [0.1484 - 0.4083] | <0.001 |
| | 25-34 | 0.0152 | 0.292 | 0.0398 | | | | 0.0000 | 0.292 | 0.0026 | | | |
| | 35-44 | 0.0076 | 0.252 | 0.0267 | | | | 0.0029 | 0.252 | 0.0067 | | | |
| | 45-54 | 0.0000 | 0.247 | 0.0318 | | | | 0.0000 | 0.247 | 0.0152 | | | |
| | 55-64 | 0.0000 | 0.198 | 0.0150 | | | | 0.0059 | 0.198 | 0.0186 | | | |
| | 65-75 | 0.0000 | 0.088 | 0.0027 | | | | 0.0000 | 0.088 | 0.0072 | | | |
| | >75 | 0.0076 | 0.088 | 0.0020 | | | | 0.0029 | 0.088 | 0.0108 | | | |
| Male | 18-24 | 0.0000 | 0.332 | 0.0151 | | | | 0.0000 | 0.332 | 0.001 | | | |
| | 25-34 | 0.0076 | 0.350 | 0.0398 | | | | 0.0029 | 0.350 | 0.0051 | | | |
| | 35-44 | 0.0000 | 0.367 | 0.0361 | | | | 0.0088 | 0.367 | 0.0194 | | | |
| | 45-54 | 0.0076 | 0.314 | 0.0238 | | | | 0.0059 | 0.314 | 0.0258 | | | |
| | 55-64 | 0.0000 | 0.216 | 0.0164 | | | | 0.0088 | 0.216 | 0.031 | | | |
| | 65-75 | 0.0000 | 0.113 | 0.0026 | | | | 0.0059 | 0.113 | 0.0162 | | | |
| | >75 | 0.0076 | 0.113 | 0.0017 | | | | 0.0000 | 0.113 | 0.0182 | | | |

| | | | | | | | | | | | | | |
|---|---|---|---|---|---|---|---|---|---|---|---|---|---|
| CSR: Class specific rate | | | | | | | | | | | | | |

In 2018, in France the mean number of daily cigarettes by daily current smokers was 13,0 cigarettes (or equivalence) by day in the general population, 14,0/day for men and 11,9 for women (Andler, R. et al., previously cited).

Daily cigarette consumption of current smokers in the in- and out-patients is shown in Table 3. Heavy daily current smokers (20 or more cigarettes a day) were two of 7 outpatients and 5 of 15 inpatients (data unavailable for 1 inpatient).

**TABLE 3 - Daily cigarette consumption of outpatient and inpatient current smokers**

| | sex | age | daily cigarette consumption |
|---|---|---|---|
| OUT1 | H | 47 | 5 |
| OUT2 | F | 37 | 5 |
| OUT3 | F | 25 | <5 |
| OUT4 | M | 27 | 5 |
| OUT5 | F | 28 | 20 |
| OUT6 | M | 55 | >=20 |
| OUT7 | F | 18 | 10 |
| IN1 | M | 49 | <5 |
| IN2 | F | 57 | >20 |
| IN3 | M | 48 | NA (transfer in ICU) |
| IN4 | M | 60 | >=20 |
| IN5 | F | 81 | >=20 |
| IN6 | M | 74 | >=20 |
| IN7 | F | 39 | 5 |
| IN8 | M | 36 | <5 |
| IN9 | M | 66 | 10 |
| IN10 | M | 31 | 5 |
| IN11 | M | 43 | 10 |
| IN12 | M | 64 | 8 |
| IN13 | M | 61 | 15 |
| IN14 | F | 61 | 20 |
| IN15 | M | 36 | 1 |

### Discussion

The present study reports for the first time a decreased prevalence of daily current smokers in both COVID19 outpatients and inpatients compared to the general population. These findings clearly support the hypothesis that current daily smoking may be a protective factor against COVID19 infection, or at least symptomatic COVID19 infection.

The Standardized Incidence Ratio of current daily smoking in COVID19 outpatients and inpatients were 0.197 [0.094 - 0.41] and 0.246 [0.148 - 0.408], respectively meaning that a significantly decreased current daily smoker rate is observed, compared to the French general population. This decrease is of 80.5% and 75.4% in outpatients and inpatients, respectively. SIR did not differ between outpatients and inpatients, suggesting that the smoking protective effect was on the whole population of symptomatic (non-severe and severe) patients.

### II. MOLECULAR DATA: role of nAChR on COVID-19 infection

The present inventors hypothesized that the cerebral nicotinic acetylcholine receptor (nAChR) may play a key role in the pathophysiology of COVID-19 infection.

Several scientific clues favor this hypothesis:
First, it is accepted that the angiotensin converting enzyme ACE2, represents the principal receptor molecule for SARS-CoV-2 (Yan, R. et al. Structural basis for the recognition of SARS-CoV-2 by full-length human ACE2. Science 367, 1444-1448, doi:10.1126/science.abb2762 (2020)). The ACE2 is expressed in the brain, in both neurons and glia, being in particular present in the brain stem and in the regions responsible for regulation of cardiovascular function including subfornical organ, paraventricular nucleus, nucleus of the tractus solitarius, and rostral ventrolateral medulla. Yet additional receptors or co-receptors are not excluded. The relationship between nicotine and ACE2 has been explored in the framework of cardiovascular and pulmonary diseases. Accordingly, in the ACE/ANG II/AT1R arm, nicotine increases the expression and/or activity of renin, ACE and AT1R, whereas in the compensatory ACE2/ANG-(1-7)/MasR arm, nicotine down regulates the expression and/or activity of ACE2 and AT2R thus suggesting a possible contribution of nAChR in ACE2 regulation. This possibility has not yet been explored in the framework of viral neuroinfections.
Second, there are strong evidences for a neurotropic action of SARS-CoV-2 infection. It has been indeed demonstrated that ß-coronaviruses, to which the SARS-CoV-2 belongs, do not limit their presence to the respiratory tract and frequently invade the CNS (Steardo, L., Steardo, L., Jr., Zorec, R. & Verkhratsky, A. Neuroinfection may potentially contribute to pathophysiology and clinical manifestations of COVID-19. Acta Physiol (Oxf), e13473, doi:10.1111/apha.13473 (2020)). This propensity has been convincingly documented for the SARS-CoV-1, MERS-CoV and the coronavirus responsible for porcine hemagglutinating encephalomyelitis (HEV 67N). In light of the high similarity between SARS-CoV-1 and SARS-CoV-2, it is quite likely that SARS-CoV-2 also possesses a similar potential. Neuroinfection has been proposed to potentially contribute to pathophysiology and clinical manifestations of COVID-19 and the neuroinvasive potential of SARS-CoV-2 suggested to play a role in the respiratory failure of COVID-19 patients (Baig, A. M., Khaleeq, A., Ali, U. & Syeda, H. Evidence of the COVID-19 Virus Targeting the CNS: Tissue Distribution, Host-Virus Interaction, and Proposed Neurotropic Mechanisms. ACS Chem Neurosci 11, 995-998, doi:10.1021/acschemneuro.0c00122 (2020)).
Third, loss of sense of smell frequently occurs in COVID-19 patients (Gane, S. B., Kelly, C. & Hopkins, C. Isolated sudden onset anosmia in COVID-19 infection. A novel syndrome? Rhinology, doi:10.4193/Rhin20.114 (2020)). Furthermore, several studies have reported that some patients infected with SARS-CoV-2 show neurologic signs such as headache (about 8%), nausea and vomiting (1%). More recently, one study on 214 COVID-19 patients further found that about 88% (78/88) among the severe patients displayed neurologic manifestations including acute cerebrovascular diseases and impaired consciousness (Ling Mao, M. W., Shanghai Chen, Quanwei He, Jiang Chang, Candong Hong, Yifan Zhou, David Wang, Yanan Li, Huijuan Jin, Bo Hu. Neurological Manifestations of Hospitalized Patients with COVID-19 in Wuhan, China: a retrospective case series study. medRxiv, doi:https://doi.org/10.1101/2020.02.22.20026500 (2020)). Based on an epidemiological survey on Covid-19, the median time from the first symptom to dyspnea was 5.0 days, to hospital admission was 7.0 days, and to the intensive care was 8.0 days. Therefore, the latency period may be enough for the virus to enter the nervous system, invade the brain stem and affect the medullary neurons of the respiratory centers.
Fourth, the nAChR is known to play a critical role in the host-pathogen interaction in the case of other well-documented viruses, such as the rabies virus (RABV).
Finally, nAChRs are present in the lung epithelium. The non-neuronal cholinergic system contributes to the regulation of cell functions such as cell-cell interaction, apoptosis, and proliferation and it is well established that human bronchial epithelial cells contain nAChRs. The airway epithelium expresses *α*3, *α*4, *α*5, *α*7, *α*9, *β*2, and *β*4 subunits for nAChRs and their contribution has been discussed in the framework of airway epithelial basal cell proliferation-differentiation and their alteration in lung cancers. These nAChRs are mentioned here as possible targets of Covid-19 infection of the lung, which would take place concomitantly with, and/or as a consequence of, the neuro-infection.

For all these reasons, the nAChR pathway is likely to be engaged in the Covid-19 inflammatory syndrome. The nervous system, through the vagus nerve, can inhibit significantly and rapidly the release of macrophage TNF, IL-1, IL-6, IL-18 and attenuate systemic inflammatory responses). This physiological mechanism, termed the 'cholinergic anti-inflammatory pathway' has major implications in immunology and in therapeutics.

Macrophages mediate physiologic control of cytokine production by auto/paracrine acetylcholine through the nAChR expressed in these cells. The profile of cytokines massively secreted following a loss of control by acetylcholine through dysregulation of macrophage nAChR are IL1, IL6, TNF and IL18. This cytokine profile has striking analogies with the cytokine storm syndrome, leading to the hyperinflammatory syndrome, that has been described in a subgroup of COVID-19 patients (Chen, G. et al. Clinical and immunologic features in severe and moderate Coronavirus Disease 2019. J Clin Invest, doi:10.1172/JC1137244 (2020)). nAChR modulation by COVID-19 might thus account for the hyperinflammatory features observed in a subgroup of COVID-19 patients, mimicking bona *fide* the macrophage activation syndrome.

Altogether, this nicotinic hypothesis contrasts with the two currently accepted views that the lung is the primary target of the virus and that ACE2 is the principal receptor of COVID-19 for its entry in cells.

The present study shows that the rates of current smoking remain below 5 % even when main confounders for tobacco consumption, i.e. age and sex, in- or outpatient status, were considered. As shown above, compared to the French general population, the COVID-19 population exhibited a significantly decreased current daily smoker rate by 80,5% for outpatients and by 75.4% for inpatients. Thus, current smoking status appears to be a protective factor against the infection by SARS-CoV-2. Although the chemistry of tobacco smoke is complex, these data are consistent with the hypothesis that its protective role takes place through direct action on various types of nAChRs expressed in neurons, immune cells (including macrophages), cardiac tissue, lungs, and blood vessels.

Moreover, they are structural evidences supporting the hypothesis that SARS-CoV-2 virus is a nicotinic agent. On a one hand, the recently reported X-ray structure of the RABV glycoprotein (G) ectodomain (Yang, F. et al. Structural Analysis of Rabies Virus Glycoprotein Reveals pH-Dependent Conformational Changes and Interactions with a Neutralizing Antibody. Cell Host Microbe 27, 441-453 e447, doi:10.1016/j.chom.2019.12.012 (2020)) shows that the region corresponding to the neurotoxin-like peptide is exposed at the G surface, in agreement with the fact that this region is part of the major antigenic region II of RABV (Bakker, A. B. et al. Novel human monoclonal antibody combination effectively neutralizing natural rabies virus variants and individual in vitro escape mutants. J Virol 79, 9062-9068, doi:10.1128/JV!.79.14.9062-9068.2005 (2005)). On another hand, the recently published cryo-EM structure of the trimeric SARS-CoV-2 spike (S) protein revealed an insertion with respect to that of SARS-CoV-1, in a loop that is disordered in the reported structure, and which has a polybasic sequence that corresponds to a furin site (Walls, A. C. et al. Structure, Function, and Antigenicity of the SARS-CoV-2 Spike Glycoprotein. Cell, doi:10.1016/j.cell.2020.02.058 (2020)). Importantly, this exposed loop of the SARS-CoV-2 S protein also contains a motif (SEQ ID NO:1: YQTQTNSPRRAR) that is homologous to that of snake neurotoxins (SEQ ID NO:2: CDGFCSSRGKR and that of the bungarotoxin SEQ ID NO:6: CDAFCSSRGKV) and to the RABV neurotoxin-like region (SEQ ID NO:3-5) (figure 1). This observation confirms the hypothesis that SARS-CoV-2 virus itself is a nAChR blocker.

**CONCLUSION*:*** Nicotine, as well as any nAChR effector ligand, is a potential preventive agent against Covid-19 infection.

Both the epidemiological/clinical evidence and the *in silico* findings suggest that Covid-19 infection is a nAChR disease that could be prevented and may be controlled by a nAChR effector ligand, in particular by nicotine. This nAChR effector ligand would then sterically or allosterically compete with the SARS-CoV-2 binding to the nAChR. This legitimates the use of a nAChR effector ligand, in particular nicotine, as a protective agent against SARS-CoV-2 infection and the subsequent deficits it causes in the CNS.

### CLINICAL TRIAL

In order to prevent the infection and the retropropagation of the virus through the CNS, a therapeutic assay has been set up to assess the role of nicotine (and other nicotinic agents) patches in hospitalized patients and in the general population against COVID-19 infection.

The primary objective of this assay is to assess the efficacy of nicotine patches in terms of prevention of unfavorable evolution in hospitalized COVID-19 patients. Its end point is when unfavorable evolution is observed. "Unfavorable evolution" is herein defined by either death or transfer to ICU or indication for ICU transfer but LATA.

The second objective is to assess the efficacy of nicotine patches in terms of survival at day 14 and day 28 post-COVID-19 detection, in particular their effect on the number of transfers to ICU, indication for ICU transfer but LATA, non-invasive ventilation or on the time to discharge.

The percent of subjects with SARS-CoV-2 detectable in nasopharyngeal sample [Time Frame: Days 3, 5, 8, 11, 15, 29] is assessed.

The SARS-CoV-2 is quantified in nasopharyngeal sample [ Time Frame: Days 3, 5, 8, 11, 15, 29 ] and in blood [ Time Frame: Days 3, 5, 8 and 11 ].

This randomized blind control trial enrols 194 patients:
**Main inclusion criteria:** Patients hospitalized in internal medicine with diagnosis of COVID-19 (either positive PCR and/or evocative thorax CT scan)
**Main exclusion criteria:** <18-year-old patients; refusal of the patients; hospitalization in ICU, LATA

They are randomized in 2 groups of 97 patients.

These two groups of patients will receive either a nicotine patch or a placebo patch on their back, once a day (single blind).

The treatment lasts 15 days, followed by 15 days of tapering, and one-month follow-up. A long-term follow up is also scheduled for 3-6 months.

The nicotine patch is NICOPATCHLIB® (21 mg, 14 mg or 7 mg) (Pierre Fabre): Transluscent, Ultra-thin, Square shape with round corners, 5,3 x 5,6 cm

The dosage will be 21 mg per day during two weeks, 14 mg/day during the third week and 7 mg/day during the fourth week.

The placebo patch is TEGADERM®: Transluscent, Ultra-thin, Square shape with round corners, 4,4 cm x 4,4 cm. A placebo patch of the exact same size of NICOPATCHLIB® may also be used.

## Claims

1. Ligand having a modulatory effect on neuronal nicotinic acetylcholine receptor (nAChR), or a pharmaceutical salt thereof, for use for preventing and/or treating viral infections due to at least one betacoronavirus.

2. Ligand having a modulatory effect on neuronal nicotinic acetylcholine receptor (nAChR), or a pharmaceutical salt thereof, for use according to claim 1, wherein the betacoronavirus is a Severe Acute Respiratory Syndrome-related coronavirus (SARS-CoV), in particular SARS-CoV-2.

3. Ligand having a modulatory effect on neuronal nicotinic acetylcholine receptor (nAChR), or a pharmaceutically acceptable salt thereof, for use according to claim 2, for preventing and/or treating the symptomatic COVID-19 disease, and advantageously severe symptomatic COVID-19 disease.

4. Ligand having a modulatory effect on neuronal nicotinic acetylcholine receptor (nAChR), or a pharmaceutically acceptable salt thereof, for use according to any one of claims 1 to 3 for preventing and/or treating acute respiratory distress syndrome.

5. Ligand having a modulatory effect on neuronal nicotinic acetylcholine receptor (nAChR), or a pharmaceutically acceptable salt thereof, for use according to any one of claims 1 to 4 for increasing the survival rate of patients.

6. Ligand having a modulatory effect on neuronal nicotinic acetylcholine receptor (nAChR), or a pharmaceutically acceptable salt thereof, for use according to any one of claims 1 to 5, wherein said nAChR effector ligand or its pharmaceutically acceptable salt is administered via the transcutaneous route.

7. Ligand having a modulatory effect on neuronal nicotinic acetylcholine receptor (nAChR), or a pharmaceutically acceptable salt thereof, for use according to any one of claims 1 to 6, wherein said nAChR effector ligand or its pharmaceutically acceptable salt thereof is administered using transdermal delivery device, advantageously using a transdermal patch.

8. Ligand having a modulatory effect on neuronal nicotinic acetylcholine receptor (nAChR), or a pharmaceutically acceptable salt thereof, for use according to any one of claims 1 to 7, wherein said nAChR effector ligand is nicotine.

9. Nicotine or a pharmaceutically acceptable salt thereof for use according to claim 8, wherein the nicotine or the pharmaceutically acceptable salt thereof is administered in a dosage of between 5 and 30 mg per 24 hours.

10. A transdermal delivery device comprising a nAChR effector ligand or a pharmaceutically acceptable salt thereof for use for preventing and/or treating viral infections due to at least one betacoronavirus, advantageously a Severe Acute Respiratory Syndrome-related coronavirus, in particular SARS-CoV-2.

11. A transdermal delivery device comprising a nAChR effector ligand or a pharmaceutically acceptable salt thereof for use according to claim 10 for preventing and/or treating symptomatic disease, more particularly severe symptomatic disease, and/or respiratory distress syndrome and/or for increasing the survival rate of patients.

12. A transdermal delivery device comprising a nAChR effector ligand or a pharmaceutically acceptable salt thereof for use according to claim 10 or 11, wherein the disease is COVID-19 disease and patients are COVID-19 patients.

13. The transdermal delivery device for use according to any one of claims 10 to 12, wherein the device is a transdermal patch.

14. The transdermal delivery device for use according to any one of claims 10 to 13, wherein the nAChR effector ligand is nicotine.

15. The transdermal delivery device for use according to any one of claims 10 to 14, wherein the device comprises between 5 and 30 mg of nicotine or a pharmaceutically acceptable salt thereof.
